(19) Europäisches Patentamt
European Patent Office
Office européen des brevets

(11) **EP 2 302 375 B1**

(12) **EUROPÄISCHE PATENTSCHRIFT**

(45) Veröffentlichungstag und Bekanntmachung des Hinweises auf die Patenterteilung:
**12.09.2012 Patentblatt 2012/37**

(51) Int Cl.:
*G01N 33/487* (2006.01)       *G01N 21/64* (2006.01)

(21) Anmeldenummer: **09012306.8**

(22) Anmeldetag: **29.09.2009**

(54) **Verfahren und Vorrichtung zum Erfassen der Stromspannungskurve einer Zelle**

Method and device for recording the current voltage curve of a cell

Procédé et dispositif de saisie de la courbe de tension de courant d'une cellule

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(43) Veröffentlichungstag der Anmeldung:
**30.03.2011 Patentblatt 2011/13**

(73) Patentinhaber: **Karlsruher Institut für Technologie**
**76131 Karlsruhe (DE)**

(72) Erfinder: **Wegner, Lars**
**76676 Graben-Neudorf (DE)**

(74) Vertreter: **Gärtner, Stephan**
**Karlsruher Institut für Technologie**
**Stabsabteilung Innovation**
**Postfach 36 40**
**76021 Karlsruhe (DE)**

(56) Entgegenhaltungen:
**EP-A1- 1 178 315       EP-A2- 1 281 961**
**DE-A1- 19 948 473       US-A1- 2003 121 778**

• **FROMHERZ P ET AL: "Voltage-sensitive fluorescence of amphiphilic hemicyanine dyes in neuron membrane" BIOCHIMICA ET BIOPHYSICA ACTA. BIOMEMBRANES, AMSTERDAM, NL, Bd. 1150, Nr. 2, 15. August 1993 (1993-08-15), Seiten 111-122, XP023352250 ISSN: 0005-2736 [gefunden am 1993-08-15]**
• **PETER FROMHERZ ET AL: "ANNINE-6plus, a voltage-sensitive dye with good solubility, strong membrane binding and high sensitivity" EUROPEAN BIOPHYSICS JOURNAL ; WITH BIOPHYSICS LETTERS, SPRINGER, BERLIN, DE, Bd. 37, Nr. 4, 9. August 2007 (2007-08-09) , Seiten 509-514, XP019586181 ISSN: 1432-1017**

**Beschreibung**

Die vorliegende Erfindung betrifft ein Verfahren und eine Vorrichtung zum Erfassen der Strom-Spannungskennlinie von Zellen.

Zellmembranen bestehen aus einer Lipiddoppelschicht, die im Wesentlichen undurchlässig ist für Ladungsträger wie z. B. Ionen. Die Leitfähigkeit einer Zellmembran wird durch die in der Membran eingelagerten Transmembranproteine vermittelt. Diese Proteine haben wichtige physiologische Funktionen, insbesondere sind sie für den Transport von Substanzen durch die Membran hindurch verantwortlich. Ermöglicht ein Transmembranprotein den Transport von geladenen Teilchen (Ionen) durch die Membran, so spricht man auch von einem Ionenkanal.

Die präzise Vermessung der Aktivität von Ionenkanälen in der Plasmamembran von Zellen und ihre gezielte Beeinflussung durch Wirkstoffe ist von elementarer Bedeutung für die pharmakologische Forschung und Diagnostik, gewinnt aber auch an Bedeutung in der Pflanzenbiotechnologie, z.B. in der Züchtungsforschung zur Identifizierung salztoleranter Sorten und in der Phytopathologie beispielsweise zur Untersuchung der Interaktion von Pathogen und Wirtspflanze. Beide Fragestellungen eignen sich für die Entwicklung Ionenkanal-basierter Messungen unter Verwendung diagnostisch relevanter Zellen oder von Zellen, die als Expressionssystem eingesetzt werden, wie z.B. Hefezellen. Die Wirkung einer Substanz auf die Ionenkanalaktivität kann durch die Vermessung der Strom-Spannungskennlinie der jeweiligen Zelle, d.h. der spannungsabhängigen Leitfähigkeit der Zellmembran, vor und nach Applikation der betreffenden Substanz quantifiziert werden.

Zur elektrophysiologischen Vermessung der Leitfähigkeit von Zellmembranen wird hauptsächlich die sog. Patch-Clamp-Technik verwendet. Hiermit können die biophysikalischen Eigenschaften der Ionenkanäle untersucht werden. In der klassischen Variante wird eine Glas-Mikropipette auf die zu vermessende Zelle aufgebracht und die Zelle wird durch Anlegen eines Unterdrucks an die Pipette angezogen, wobei zwischen Pipettenende und der Zellmembran ein dichter Verschluss, der sog. Seal, entsteht. Dieser Seal muss entsprechend fest sein, sodass ein elektrischer Widerstand am Seal von mehr als 1 GOhm entsteht (Gigaseal). Mit Hilfe von Elektroden, die im Inneren der Pipette und außerhalb der Pipette angebracht sind, kann eine Spannung angelegt werden und die Leitfähigkeit der Zellmembran gemessen werden.

Die Patch-Clamp-Technik in der klassischen Form hat den Nachteil, dass sie einen erheblichen experimentellen Aufwand darstellt. Das Patch-Clamp Experiment wird unter manueller Kontrolle unter einem Mikroskop durchgeführt. Dies erfordert ein qualifiziertes Personal. Die Erfolgsrate des Gigaseals hängt, neben dem Geschick des Durchführenden, zum einen von der Qualität der Mikropipette ab, zum anderen von der Zelle, die nicht immer uneingeschränkt zugänglich ist. Beispielsweise sind pflanzliche Zellen oder Hefen, die häufig als Modellorganismus verwendet werden, von einer Zellwand umgeben, die vor dem Patch-Clamp-Experiment enzymatisch von der Zellwand befreit werden müssen, da sich ansonsten kein Gigaseal ausbildet. Auch tierische Zellen, sofern sie nicht aus einer Zellkultur stammen, müssen oft einer enzymatischen Vorbehandlung unterzogen werden.

Abhängig davon, ob nach dem Aufsetzen der Mikropipette der Membranbereich aus der Zelle herausgelöst oder an der intakten Zelle gemessen wird, werden bestimmte Konfigurationen in der Patch-Clamp-Technik unterschieden.

In der einfachsten Variante der Patch-Clamp-Technik ist die Mikropipette auf einen Bereich der Zellmembran aufgesetzt, in dem sich nur ein Ionenkanal befindet (sog. Cell-Attached-Konfiguration). Hierbei sind die gemessenen Transmembranströme sehr schwach und geben keine Auskunft über die Ströme, die durch die gesamte Zelloberfläche fließen.

Zur Messung der Ströme, die über die gesamte Membran fließen, kann die sog. Whole-Cell-Konfiguration verwendet werden. Hierzu wird der an der Pipette haftende Membranbereich geöffnet, während der Gigaseal intakt bleibt. Zwischen dem Inneren der Pipette und dem Inneren der Zelle besteht nun eine Kontinuität, während beide gegen die Außenlösung durch den hohen Widerstand des Gigaseals isoliert sind. Nachteil der Whole-Cell-Konfiguration ist die Invasivität, d.h. die Zelle wird zerstört und kann nicht für Folgeexperimente verwendet werden. Ein weiterer Nachteil der Patch-Clamp-Technik beider Konfigurationen ist die begrenzte Automatisierbarkeit des Experiments hinsichtlich eines Hochdurchsatzverfahrens.

Eine Automatisierungsvariante der Patch-Clamp-Technik wird in der Patentschrift EP 0980523 B1 beschrieben. Hierzu ist eine umfangreiche Elektroden-Positioniervorrichtung notwendig, die ein Steuerungssystem umfasst, mit welcher die Pipettenspitze auf die Zelle zu positioniert wird. Eine menschliche Bedienperson ist hier nicht notwendig, jedoch bleiben die oben beschriebenen Nachteile der klassischen Patch-Clamp-Technik bestehen.

In US Patent 6,268,168 wird eine zellphysiologische Arbeitsstation für eine automatisierte Datenerfassung beschrieben. Mit dieser Apparatur können auch elektrophysiologische Untersuchungen gemacht werden, jedoch nur auf Basis einer Zwei-Elektroden-Spannungsklemme, die mit der Einführung von Mikrokapillaren in die Zelle verbunden ist. Neben dem hohen technischen Aufwand durch den Einsatz von Mikromanipulatoren führt diese Technik zu einer hohen Fehlerquote bei ungenügender Abdichtung der Einstichstelle oder Fehlplatzierung der Elektrodenspritze. Zudem ist das Einführen einer Mikrokapillare stets mit einem erheblichen Eingriff in die Integrität der Zelle verbunden, was zu Messartefakten führen kann.

US-Patent 6,699,697 offenbart ein planares Array mit einzelnen Messzellen zur Durchführung der Patch-Clamp-Technik in der Cell-Attached Konfiguration. Die Messzellen sind in einem Silikon-Polymersubstrat aufgebracht und umfassen eine Öffnung, durch welches die Zellen angesaugt werden, zwecks Ausbildung eines Seals von mindestens 100 MOhm. Diese Vorrichtung stellt eine Automatisierungsvariante der Patch-Clamp-Technik dar, weil mehrere Zellen gleichzeitig vermessen werden können, jedoch bestehen auch hier die oben genannten Nachteile der Patch-Clamp-Technik.

In EP 1283985 B1 wird ein Verfahren und eine Vorrichtung offenbart, mit der die Patch-Clamp Konfigurationen dergestalt abgeändert werden, dass die zu vermessenden Zellen in das Lumen einer Kapillare eingeschleust werden und nicht am Pipettenende angesaugt werden. Der schwierige Schritt des Orientierens der Pipette auf die Zelle unter optischer Kontrolle wird somit überflüssig. Die Zelle wird in der Kapillare, deren Innendurchmesser an mindestens einer Stelle kleiner ist als der Zellaußendurchmesser, festgeklemmt, wodurch sich ein Seal von mindestens 10 GOhm ausbilden muss. Dies erfordert eine sehr reine Innenoberfläche der Kapillare. Des Weiteren werden die Zellen auf einer Seite permeabilisiert, wodurch die Zelle für weitere Experimente unbrauchbar gemacht wird und der Zellinhalt durch Pipettiermedium ausgetauscht wird. Dadurch wird die intrazelluläre Konzentration an Ionen, Proteinen, Nukleinsäure, etc. im Vergleich zur intakten Zelle verändert, was zu Messartefakten führen kann. Bei der Erfassung der Strom-Spannungskurve nach diesem Verfahren erhält man einen unspezifischen Summenstrom, der abzüglich der Kriechströme am Gigaseal durch die Zelle fließt.

EP1178315 offenbart eine Patch-Clamp-Vorrichtung mit einer Kapillare, die einen in der Mitte liegenden konisch zulaufendem Bereich und Elektroden an ihren beiden Enden aufweist. Zusätzlich kann eine fluoreszenzoptische Messung vorgesehen sein.

Die Verwendung von spannungssensitiven Fluoreszenzfarbstoffen zum Dotieren von Zellen in einer Patch-Clamp-Konfiguration wird von P. FROMHERZ et al. in BIOCHIMICA ET BIOPHYSICA ACTA 1150 (1993), Seiten 111-122, beschrieben.

Ausgehend hiervon ist es die Aufgabe der Erfindung, ein Verfahren und eine Vorrichtung vorgeschlagen, um die Strom-Spannungskennlinie einer Zelle aufzuzeichnen, welche die Nachteile des Stands der Technik zumindest teilweise überwinden. Eine weitere Aufgabe der Erfindung ist es eine Anordnung mehrerer erfindungsgemäßer Vorrichtungen vorzuschlagen, um das Verfahren weitgehend zu automatisieren.

Die vorliegende Erfindung löst die Aufgabe durch das in Anspruch 1 beanspruchte Verfahren, die in Anspruch 9 beanspruchte Vorrichtung und die in Anspruch 12 beanspruchte Anordnung. Bevorzugte Ausführungsformen sind in den rückbezogenen Ansprüchen beschrieben.

Die vorliegende Erfindung basiert auf dem Prinzip, dass eine Zelle durch ein externes elektrisches Feld polarisiert wird. Die daraus resultierende Aufladung der Membran wird mit hoher räumlicher Auflösung optisch verfolgt. Des Weiteren wird angenommen, dass eine homogene Verteilung der Ionenkanäle über die Zelloberfläche vorliegt. Die Aufladung der Membran im elektrischen Feld erzeugt einen transzellulären Summenstrom, der auf Ionentransport durch die Zellmembran zurückgeht.

Die Erfindung umfasst folgende Verfahrensschritte:

a) Bereitstellen einer Zelle, die mittels eines spannungssensitiven Fluoreszenzfarbstoffs dotiert ist,
b) Bereitstellen einer Messvorrichtung umfassend:

- eine Kapillare mit einem proximalen und einem distalen Ende sowie einen dazwischen liegenden konisch zulaufenden Bereich,
- eine erste Elektrode, die auf der proximalen Kapillarseite angeordnet ist,
- eine zweite Elektrode, die auf der distalen Kapillarseite angeordnet ist und
- ein fluoreszenzoptisches Detektionssystem zur Detektion des Membranpotentials der Zelle im elektrischen Feld in Form eines optischen Signals,

c) Einführen der dotierten Zelle in das Lumen der Kapillare und Fixieren der Zelle in dem konisch zulaufenden Bereich der Kapillare mittels Druckperfusion bis sich durch den Andruck der Zelle an die Kapillarwand ein elektrischer Klemmwiderstand zwischen dem distalen und proximalen Ende der Kapillare ausbildet, bevorzugter weise beträgt der Klemmwiderstand 50 bis 10000 MOhm,
d) Anlegen einer Spannung zwischen den beiden Elektroden und Aufzeichnen des transzellulären Gesamtstroms $I_{ges}$, der durch die mit der Zelle verstopfte Kapillare fließt,
e) Messen des lokal und zeitlich aufgelösten Membranpotentials $U_{lok}$ der Zelle im elektrischen Feld zwischen den Elektroden mit Hilfe des fluoreszenzoptischen Detektionssystems,
f) Berechnung eines lokalen Stroms $I_{lok}$ mit Hilfe des gemessenen transzellulären Gesamtstroms $I_{ges}$ aus Schritt d) und des gemessenen lokalen Membranpotentials $U_{lok}$ aus Schritt e),
g) Wiederholen der Schritte d) bis f), wobei die in Schritt d) angelegte Spannung variiert wird, und Aufzeichnung der resultierenden lokalen Ströme $I_{lok}$ gegen die lokalen Spannungen $U_{lok}$ aus Schritt e), wodurch nicht-invasiv die Strom-Spannungskurve der untersuchten Zelle erhalten wird.

In einer bevorzugten Ausführung erfolgt die Berechnung eines lokalen Stroms $I_{lok}$ aus Schritt f) des erfindungsgemäßen Verfahrens anhand zweier Zusammenhänge. Erstens wird der lokale Beitrag des Stroms $I_{lok}$ zum gemessenen lokalen Membranpotential $U_{lok}$ proportional gemäß des Ohmschen Gesetzes zugeordnet. Zweitens wird ein Bezug des lokalen Stroms $I_{lok}$ zum transzellulären Gesamtstrom $I_{ges}$ hergestellt durch Aufsummieren oder Integration der Teilströme über die Membranflächen, z.B.

$$\sum I_{lok} = I_{ges}.$$

Sowohl der transzellulären Gesamtstrom $I_{ges}$ als auch die lokalen Membranpotentiale $U_{lok}$ sind durch die Messung zugänglich. Die Zuordnung eines lokalen Stroms $I_{lok}$ zu einem lokalen Membranpotential $U_{lok}$ kann beispielsweise auf Basis eines umformulierten Ohmschen Gesetzes erfolgen, gemäß

$$I_{lok} = G(U) * (U_{lok} - U_0),$$

wobei $I_{lok}$ der lokale Beitrag des Stroms, $G(U)$ die Leitfähigkeit der Zelle in Abhängigkeit der angelegten Spannung, $U_{lok}$ das lokale Membranpotential und $U_0$ das Membranpotential bei dem der Strom $I_{lok}$ den Wert 0 einnimmt. Bei der Annahme der Proportionalität ist zu beachten, dass die Leitfähigkeit G bzw. der Widerstand R (G=1/R) eine Funktion der angelegten äußeren Spannung ist. Vorteilhaft ist diese mathematische Prozessierung der lokalen Ströme $I_{lok}$, da sie interpretierbare Daten zu den zellulären Membrantransportprozessen liefert.

Durch konsekutive Spannungspulse unterschiedlicher Amplitude und Aufzeichnung der berechneten lokalen Ströme $I_{lok}$ gegen die lokalen Membranpotentiale $U_{lok}$ wird die Strom-Spannungskurve der Zelle aufgenommen.

Das Dotieren der Zelle mit einem spannungssensitiven Fluoreszenzfarbstoff kann prinzipiell auf zwei dem Fachmann bekannten Weisen durchgeführt werden. Zum einen können für das Experiment Zellen verwendet werden, die einen Protein-basierten optischen Spannungssensor intrinsisch exprimieren. Zum anderen können die Zellen mit einem Fluoreszenzfarbstoff vor dem Experiment inkubiert werden.

Zur Klasse der spannungssensitiven Fluoreszenzfarbstoffe zählen insbesondere Styryl-Farbstoffe, Oxonole und vorzugsweise Hemicyanine. Diese amphiphilen Farbstoffe lagern sich beim Inkubieren in die Membran der Zelle ein (intercalieren) und zeigen eine Verschiebung des Fluoreszenzspektrums im elektrischen Feld. Besonders bevorzugt wird das Hemicyanin Annine-6 verwendet, da dieser Farbstoff keinen Einfluss auf die Kanalaktivitäten der Zelle hat.

Das Positionieren der dotierten Zelle in das Lumen der Kapillare wird mittels bekannter mikrofluidischer Methoden durchgeführt. In einer bevorzugten Ausführung wird ein mikrofluidisches System mit einer Zellsuspension durchspült, bis eine Zelle in den konischen Bereich vordringt und den weiteren Durchfluss blockiert. Durch Erhöhen des Fluiddrucks, kann die Zelle stärker an die Kapillarwand angepresst werden, wodurch sich der elektrische Widerstand zwischen Zellwand oder Zellmembran erhöht.

Zentraler Vorteil bei dem vorgestellten Verfahren ist, dass Protoplasten oder pflanzliche Zellen, welche eine Zellwand besitzen, direkt und ohne enzymatische Vorbehandlung verwendet werden können. Ein sog. Gigaseal wie es im klassischen Patch-Clamp-Verfahren notwendig ist kann nur mit Zellen ohne Zellwand erreicht werden. Bei dem hier vorgestellten Verfahren kommt man mit einem niedrigeren Klemmwiderstand von 50 bis 10000 MOhm aus. Bevorzugt wird ein Klemmwiderstand von 100 bis 1000 MOhm verwendet.

In einer bevorzugten Ausführung des Verfahrens wird der elektrische Klemmwiderstand zwischen Zellwand oder Zellmembran und Kapillare über Impedanzmessungen ermittelt, damit der Leckstrom quantifiziert werden kann. Es wird ein Wechselfeld unterschiedlicher Frequenz im Bereich von 1 Hertz bis 10000 Hertz angelegt, und die phasenverschobene Stromantwort wird aufgezeichnet. Anhand eines Ersatzschaltbildes, das die Messsituation wiedergibt, können die beteiligten Widerstände und Kapazitäten berechnet werden; hierbei handelt es sich um ein dem Fachmann geläufiges Standardverfahren. Die Differenz zwischen dem gemessenen Gesamtstrom und dem Leckstrom ergibt den transzellulären Strom.

Wird an die zu vermessende Zelle, die in der Kapillare mit dem erforderlichen Mindestwiderstand festgeklemmt wurde, eine Spannung angelegt, so kommt es im elektrischen Feld zu einer entgegen gesetzten, nicht-homogenen Aufladung beider Hemisphären der Zelle. Eine Hemisphäre ist der Anode zugewandt, die andere Hemisphäre ist der Kathodenseite zugewandt. Mit Hilfe der Fluoreszenzmessung kann die elektrische Potentialverschiebung an den Membranen der Zellen mit guter räumlicher und zeitlicher Auflösung erfasst werden.

Das lokale Membranpotential kann durch Auswertung der ortsaufgelösten Fluoreszenzintensität berechnet werden. Man erhält eine Potenzialverteilung in Abhängigkeit vom Winkel gegen die Achse, die durch die Zellpole verläuft.

Es besteht, je nach Aufgabenstellung, bei der Messung prinzipiell die Möglichkeit, den Strom aufzuzeichnen, der durch eine einzige Hemisphäre der Zelle fließt oder über alle beiden Hemisphären der Zelle fließt.

Besonders vorteilhaft bei diesem Messverfahren ist die Anwendbarkeit auf eine Vielzahl von Zelltypen, die ohne spezielle enzymatische Vorbehandlung eingesetzt werden können, da ein Gigaseal nicht notwendig ist. Dementsprechend sind Direktmessungen mit Pflanzenzellen und daraus hergestellten sog. Protoplasten aber auch mit tierischen Zellen möglich. Im Zusammenhang mit der vorliegenden Erfindung umfasst der Begriff Zelle prokaryontische und eukaryontische Zellen, aber auch Zellorganellen, wie z.B. Mitochondrien, Endoplasmatisches Retikulum oder Chloroplasten, aber auch künstliche Lipidvesikel. Besonders bevorzugt werden Hefezellen, insbesondere Saccharomyces *cerevisiae* oder *Pichia pastoris,* verwendet, da diese häufig als Modellorganismen herangezogen werden.

In einer vorteilhaften Ausführung wird für das Verfahren eine Zelle mit annähernd sphärischer Gestalt verwendet. Somit kann die Berechnung der Membranfläche, durch welche die lokalen Ströme fließen, über eine Kugelfläche modelliert werden.

Die erfindungsgemäße Messvorrichtung umfasst:

- eine Kapillare mit einem proximalen und einem distalen Ende sowie einen dazwischen liegenden konisch zulaufenden Bereich, dessen Innendurchmesser an mindestens einer Stelle kleiner ist als der Außendurchmesser der Zelle,
- eine mikrofluidische Vorrichtung, die in Kontakt mit dem proximalen Ende der Kapillare ist, zum Positionieren der Zelle in der Kapillare,
- eine erste Elektrode, die auf der proximalen Kapillarseite angeordnet ist,
- eine zweite Elektrode, die auf der distalen Kapillarseite angeordnet ist,
- ein Messinstrument zur Messung des zwischen beiden Elektroden fließenden Gesamtstroms bei angelegter Spannung,

**dadurch gekennzeichnet, dass** die Vorrichtung ferner ein fluoreszenzoptisches Detektionssystem umfasst, um das lokal und zeitlich aufgelöste Membranpotential der Zelle im elektrischen Feld in Form eines optischen Signals zu messen.

Die mikrofluidische Vorrichtung zum Positionieren der Zelle in der Kapillare umfasst mindestens einen Zulauf, durch welchen die Zellsuspension direkt in die Kapillare eingespült wird. Dieser Zulauf kann mit Druck beaufschlagt werden, sodass die Zelle im konisch verjüngten Bereich der Kapillare festgeklemmt werden kann.

In einer weiteren Ausführung kann die mikrofluidische Vorrichtung zum Positionieren der Zelle mehrere Zuläufe umfassen, wobei mindestens einer der Zuläufe mit einer Druckströmung beaufschlagt werden kann, welcher die Zelle in die Kapillare transportiert und dort mit einer Mindestdruckkraft gegen die Wandung der Kapillare gepresst wird. Die Zellsuspension kann aus einem Seitenkanal in den Druckstrom zugeführt werden, wobei der Seitenkanal so angeordnet ist, dass der Druckstrom die Zellen aus der Suspension in die Kapillare einspült. Es ist dem Fachmann ersichtlich, dass diese Fluidkanalstruktur entweder manuell bedient werden kann oder aber voll automatisierbar ist.

Außerdem umfasst die erfindungsgemäße Vorrichtung eine Auswertevorrichtung zur rechnerischen Auswertung der lokalen Ströme durch die Zellmembran aus den gemessenen Membranpotentialen. Die Auswertevorrichtung umfasst Algorithmen, mit welchen die optisch gemessenen Potentialdifferenzen an den Membranflächen den transzellulären Strömen, bzw. die lokalen Membranpotentiale den lokalen Strömen, zugeordnet werden. In einer weiteren bevorzugten Ausführung umfasst die Auswertevorrichtung einen Algorithmus, mit welchem zusätzlich die Winkelabhängigkeit der lokalen Ströme berücksichtigt wird. Mit diesen beiden Mess- bzw. Korrekturdaten werden die Inhomogenitäten der Membranaufladung im elektrischen Feld berücksichtigt und die lokalen Ströme berechnet.

Insbesondere umfasst die Auswertevorrichtung ein Modul, mit welchem die berechneten lokalen Ströme gegen die lokalen Membranpotentiale aufgezeichnet werden. Je nach Art der Interpretation der Strom-Spannungskurve können hierzu einfache graphische Darstellungen oder bildgebende Verfahren zur Anwendung kommen. Diese Möglichkeiten sind dem Fachmann geläufig.

Die Erfindung betrifft weiterhin eine mikrofluidische Anordnung zur Messung der Strom-Spannungskurve von Zellen, mit welcher mehrere Zellen gleichzeitig vermessen werden können. Diese Anordnung umfasst eine Mehrzahl an erfindungsgemäßen Messvorrichtungen zur Erfassung von Strom-Spannungskurven von Zellen, die mit spannungssensitiven Fluoreszenzarbstoffen dotiert wurden. Die einzelnen Kapillaren sind mit einer mikrofluidischen Verteilervorrichtung an ihrer proximalen Seite verbunden. Die Funktion der Verteilervorrichtung ist das Verteilen der zu vermessenden Zellen in die jeweiligen Messkapillaren. Das Beschicken der Kapillaren mit Zellen erfolgt entweder seriell, d.h. die Zellen werden aus einer Zellsuspension nacheinander in die einzelnen Kapillaren gespült, oder es erfolgt parallel, dies bedeutet ein gleichzeitiges Beschicken mehrerer Messkapillaren mit Zellen. Die Parallelanordnung der Kapillaren ist vorteilhaft, wenn beispielsweise unterschiedliche Zelltypen aus verschiedenen Kulturen gleichzeitig vermessen werden sollen.

Bei der mikrofluidischen Anordnung kann auf eine Mehrzahl von fluoreszenzoptischen Detektionssystemen verzichtet werden, wenn das fluoreszenzoptische Detektionssystem relativ zu den Kapillaren bewegt wird, sodass es die einzelnen Zellen, die in den Kapillaren festgeklemmt wurden, individuell vermessen kann.

In einer bevorzugten Ausführung umfasst die mikrofluidische Verteilervorrichtung einen oder mehrere Zuläufe für die Zellsuspensionen, abhängig davon, ob eine serielle oder parallele Verteilung der Zellen gewünscht ist, sowie Kanalstrukturen, die mit einer Druckströmung beaufschlagt werden können. Diese Druckströmung hat die Aufgabe, die Zellen aus Zellsuspensionen mitzunehmen, um sie in die Kapillaren einzuschleusen und in der Verjüngung festzupressen. Dementsprechend verläuft die Druckströmung in der Kapillare von proximaler nach distaler Richtung. Die Zuläufe sind zu diesem Zweck als Seitenkanäle zur Kanalstruktur orientiert, die in die Kanalstruktur mit der Druckströmung einmünden, sodass die Zellen von der Druckströmung mitgenommen und an ihre Zielposition in der Kapillare transportiert werden.

In einer weiteren bevorzugten Ausführung ist die mikrofluidische Anordnung rotierbar gelagert, damit durch die Drehbewegung eine Fliehkraft entsteht, die als Antrieb für die Druckströmung durch die Kanalstruktur dient. Es ist in dieser Ausführung ersichtlich, dass die Kapillaren derart orientiert sind, dass die proximale Seite tendenziell in Richtung der Rotationsachse orientiert ist und das distale Ende tendenziell von der Rotationsachse abweisend orientiert ist. Vorteilhaft bei dieser Ausführung ist, dass keine Pump- oder Saugvorrichtung notwendig sind, die Zellen erfahren dieselbe Fliehkraft, wie die Transportflüssigkeit. Dies kann sich vorteilhaft beim Festklemmen der Zelle in der Messkapillare auswirken.

Die Erfindung wird im Folgenden mit Ausführungsbeispielen und folgenden Figuren erläutert.

**Fig. 1** Positionierung einer Zelle in einer Kapillare und Elektrodenanordnung

**Fig. 2** Auslenkung des Membranpotentials der Zelle im elektrischen Feld und transzellulärer Strom.

Beispiel 1: Eine Zellsuspension (z.B. Hefezellen, aus Pflanzenzellen gewonnene Protoplasten) wird entsprechend den Herstellerangaben für 7 Minuten in einem Annine-6 haltigen Puffermedium inkubiert. Ein Aliquot der gefärbten Zellen **1** wird in den Schaft einer vorne konisch zulaufenden Glaskapillare **2** (maximaler Innendurchmesser des Schaftes: 0,8 mm, minimaler Spitzendurchmesser: 10 $\mu$m) eingefüllt. Die Spitze taucht in eine mit dem gleichen Medium gefüllte Küvette ein. Daraufhin wird am Einfüllstutzen eine Spritze mit Lühr-Anschluss angeflanscht, mit der ein leichter Überdruck (wenige mbar) angelegt werden kann. Mit der Druckströmung **6** werden Zellen **1,** deren Durchmesser größer ist als die Öffnung der Kapillarspitze in die Kapillarspitze mitgeführt. Sobald eine Zelle **1** im konischen Bereich so positioniert wurde, dass der Auslauf verstopft ist, kommt der Fluss zum Stillstand. Durch den anliegenden Druck wird die Zelle **1** unter leichter Deformierung gegen die Wandung der Kapillare **2** gepresst und ein hoher elektrischer Widerstand erzeugt, der einen Wert zwischen 50 und 10000 Megaohm erreicht.

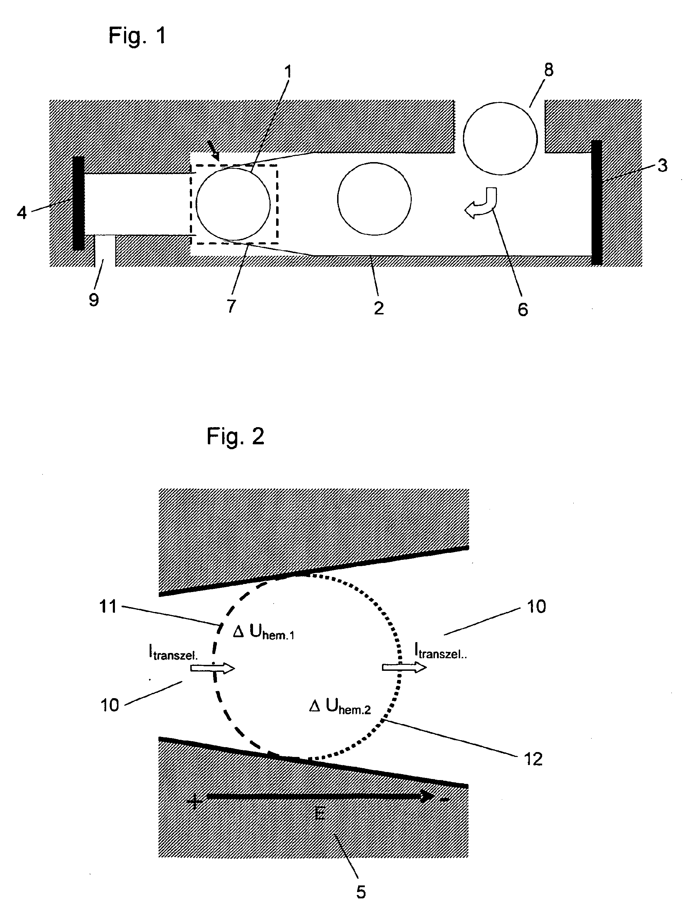

Fig. 1 zeigt schematisch eine Zelle **1,** die in der Kapillare **2** festgeklemmt wurde. Der Schaft der Kapillare **2** enthält auf der proximalen Seite einen chlorierten Silberdraht (erste Elektrode **3),** der mit einem patch-clamp Verstärker verbunden ist. Eine zweite Elektrode **4** ist auf der distalen Seite der Pipettenspitze und ebenfalls mit dem Verstärker verbunden. Zwischen beiden Elektroden kann ein elektrisches Feld **5** angelegt werden, und der dadurch ausgelöste elektrische Strom kann aufgezeichnet werden. Dieser Strom bildet die Summe aus dem transzellulären Strom **10** und dem Leckstrom, der die Zelle **1** entlang der Wandung umfließt.

Um den Abdichtwiderstand zwischen Zelle **1** und Wand zu bestimmen und damit den Leckstrom quantifizieren zu können, werden zunächst Impedanzmessungen durchgeführt. Hierzu wird ein Wechselfeld unterschiedlicher Frequenzen im Bereich von 1-10000 Hertz angelegt, und die phasenverschobene Stromantwort wird aufgezeichnet. Anhand eines Ersatzschaltbildes, das die Messsituation wiedergibt, können die beteiligten Widerstände und Kapazitäten berechnet werden. Im zweiten Versuchsabschnitt werden kurze, rechteckförmige Spannungspulse angelegt, und die Stromantwort wiederum aufgezeichnet. Der Anteil des Leckstromes am Gesamtstrom kann nun rechnerisch bestimmt werden. Die Differenz ist identisch mit dem transzellulären Strom **10.**

Die elektrische Messung wird durch eine optische Messung des Membranpotenzials in beiden Hemisphären der Zelle **1** ergänzt. Die Messung wird unter einem inversen Mikroskop so durchgeführt, dass die vermessene Zelle **1** ständig beobachtet werden kann. Wie in **Fig. 2** dargestellt kommt es im elektrischen Feld **5** zu einer entgegen gesetzten, nicht-homogenen Aufladung beider Hemisphären **11,12 ,** wobei die der Anode zugewandte Hemisphäre **11** hyperpolarisiert wird (negative Auslenkung des Membranpotentials um $\Delta U_{hem.1}$) und die zur Kathode exponierte Hemisphäre **12** wird dagegen depolarisiert (positive Auslenkung des Membranpotentials um $\Delta U_{hem.2}$). Das lokale Membranpotential U ($\alpha$) kann durch Auswertung der ortsaufgelösten Fluoreszenzintensität berechnet werden. Man erhält eine Potenzialverteilung in Abhängigkeit vom Winkel gegen die Achse, die durch die Zellpole verläuft. Diese Abhängigkeit kann beispielsweise mit der folgenden Annäherung beschrieben werden für den Fall, dass der Widerstand R konstant ist:

Der Spannungsverlauf wird als Funktion des Winkels $\alpha$ U($\alpha$) beschrieben, wobei an den Polen der Winkel 0° betragen soll. Ist R eine Konstante, so gilt für den Strom die gleiche Winkelabhängigkeit, also:

$$R = \frac{U(\alpha)}{I(\alpha)} = \frac{U^\circ f(\alpha)}{I^\circ f(\alpha)} \quad ; \quad R = 1/G$$

$U^\circ$ und $I^\circ$ sind Spannung und Strom an der Pol-Position ($\alpha=0$). $f(\alpha)$ ist z.B. $\cos(\alpha)$. Der transzelluläre Strom $I_{ges}$ **10** ist ein Messwert, der mit dem $I^\circ$ in folgende Beziehungen gesetzt wird:

$$I_{ges} = \int_{-\pi/2}^{\pi/2} f(\alpha)d\alpha * I^\circ \qquad \text{und da } U^\circ = R*I^\circ: \qquad R = \frac{U^\circ}{I_{ges}} \int_{-\pi/2}^{\pi/2} f(\alpha)d\alpha$$

In einer dreidimensionalen Annäherung wird der Winkel $\alpha$ (in der Bildebene) durch einen zweiten Winkel $\beta$ senkrecht zur Bildebene ergänzt, und es gilt:

$$R = \frac{U^\circ}{I_{ges}} \int_{-\pi/2}^{\pi/2}\!\!\int f(\alpha, \beta)d\alpha d\beta$$

Mit den beiden Winkeln $\alpha$ und $\beta$ lässt sich die jeweilige Halbkugel vollständig beschreiben.

Das experimentelle Vorgehen liefert einen transzellulären Strom sowie die dadurch erzeugten ortsaufgelösten Membranpotentiale in beiden Hemisphären der Zelle **1**. Eine mathematische Prozessierung der Daten ist erforderlich, um hieraus Strom-Spannungskurven zu generieren, da die Rohdaten hinsichtlich der Membrantransport-Prozesse nicht interpretierbar sind. Unter der Annahme, dass der Strom der gleichen räumlichen Verteilung folgt wie die Spannung, da die Eigenschaften der Zellmembran homogen angenommen werden, kann der lokale Beitrag des Stromes zum transzellulären Strom berechnet und zum lokal gemessenen Membranpotential in Beziehung gesetzt werden. Durch konsekutive Spannungspulse unterschiedlicher Amplitude kann die Strom-Spannungskurve der Zellmembran aufgezeichnet werden.

Beispiel 2: Zunächst wird die Strom-Spannungskurve einer Hefezelle, in der ein bestimmter humaner Kalium-Kanal überexprimiert wurde unter den Bedingungen, wie in Beispiel 1 beschrieben, vermessen. Dann wird ein potentieller Wirkstoff durch Medientausch in der Kapillare **2** bzw. in der mikrofluidischen Struktur zugegeben und die Messung wiederholt. Die Differenz der beiden Strom-Spannungskurven erlaubt eine Interpretation zur Wirksamkeit und zum Wirkmechanismus der Substanz.

Beispiel 3: Alternativ zum Beispiel 1 wird ein Aliquot der mit Annine-6 eingefärbten Zellen **1** wird in eine mikrofluidische Struktur mit einem Zulauf **8** und einem Ablauf **9,** sowie einer dazwischen liegenden konisch geformten Kapillare **2** gemäß **Fig. 1** gegeben. Die Zellen **1** werden von der Druckströmung **6** in die Engstelle **7** der Kapillare **2** positioniert, in welcher die Zellen **1** stecken bleiben. Die beiden Elektroden **3,4** sind beiderseits der Engstelle **7** in der mikrofluidischen Struktur positioniert. Messung und Auswertung erfolgen ähnlich wie in Beispiel 1 beschrieben.

**Bezugszeichenliste**

| | |
|---|---|
| 1 | Zelle |
| 2 | Kapillare |
| 3 | Erste Elektrode |
| 4 | Zweite Elektrode |
| 5 | Elektrisches Feld |
| 6 | Druckströmung |
| 7 | Engstelle |
| 8 | Zulauf |
| 9 | Ablauf |
| 10 | Transzellulärer Strom |

11    Hyperpolarisierte Zellmembran

12    Depolarisierte Zellmembran

**Patentansprüche**

1. Verfahren zur nicht-invasiven Messung der Strom-Spannungskurve einer Zelle **(1),** umfassend folgende Schritte:

    a) Bereitstellen einer Messvorrichtung umfassend:

        - eine Kapillare **(2)** mit einem proximalen und einem distalen Ende sowie einem dazwischen liegenden konisch zulaufenden Bereich,
        - eine erste Elektrode **(3),** die auf der proximalen Kapillarseite angeordnet ist,
        - eine zweite Elektrode **(4),** die auf der distalen Kapillarseite angeordnet ist,
        - ein fluoreszenzoptisches Detektionssystem zur Detektion eines Membranpotentials einer Zelle **(1)** im elektrischen Feld **(5)** in Form eines optischen Signals,

    b) Bereitstellen einer Zelle **(1),** die mittels eines spannungssensitiven Fluoreszenzfarbstoffs dotiert ist,
    c) Einführen der zu vermessenden Zelle **(1)** in das Lumen der Kapillare **(2)** und Fixieren der Zelle in dem konisch zulaufenden Bereich der Kapillare **(2)** mittels einer Druckströmung **(6),** bis sich durch den Andruck der Zelle **(1)** an die Kapillarwand ein elektrischer Klemmwiderstand zwischen dem distalen und dem proximalen Ende der Kapillare **(2)** ausbildet,
    d) Anlegen einer Spannung U zwischen den beiden Elektroden **(3,4)** und Aufzeichnen des transzellulären Gesamtstroms $I_{ges}$ **(10),** der durch die mit der Zelle **(1)** verstopfte Kapillare **(2)** fließt,
    e) Messen des lokal und zeitlich aufgelösten Membranpotentials $U_{lok}$ der Zelle **(1)** im elektrischen Feld **(5)** zwischen den Elektroden **(3,4)** mit Hilfe des fluoreszenzoptischen Detektionssystems,
    f) Berechnung eines lokalen Stroms $I_{lok}$ mit Hilfe des gemessenen transzellulären Gesamtstroms $I_{ges}$ **(10)** aus Schritt d) und des gemessenen lokalen Membranpotentials $U_{lok}$ aus Schritt e),
    g) Wiederholen der Schritte d) bis f), wobei die in Schritt d) angelegte Spannung variiert wird, und Aufzeichnung der resultierenden lokalen Ströme $I_{lok}$ gegen die lokalen Membranpotentiale $U_{lok}$ aus Schritt e), wodurch die Strom-Spannungskurve erhalten wird.

2. Verfahren nach Anspruch 1, wobei die Berechnung des lokalen Stroms $I_{lok}$ in Schritt f) durch

    - Zuordnung des lokalen Beitrags des Stroms $I_{lok}$ zum jeweils gemessenen lokalen Membranpotential $U_{lok}$ aus Schritt e) gemäß des Ohmschen Gesetzes und
    - in Bezug stellen zum transzellulären Gesamtstrom $I_{ges}$ **(10)** durch Integration oder Aufsummieren der Teilströme, erhalten wird.

3. Verfahren nach einem der vorgenannten Ansprüche, wobei der elektrische Klemmwiderstand zwischen dem distalen und proximalen Ende der Kapillare **(2)** mittels Impedanzspektroskopie erfasst wird.

4. Verfahren nach einem der vorgenannten Ansprüche, wobei der elektrische Klemmwiderstand zwischen 50 und 10000 MOhm liegt.

5. Verfahren nach einem der vorgenannten Ansprüche, wobei die zwischen den Elektroden **(3,4)** angelegte Spannung in Pulsen variiert wird.

6. Verfahren nach einem der vorgenannten Ansprüche, wobei der spannungssensitive Fluoreszenzfarbstoff ein Membranfarbstoff aus der Gruppe der Oxonole, Styryle oder Hemicyanine ist.

7. Verfahren nach Anspruch 6, wobei der spannungssensitive Membranfarbstoff Annine-6 ist.

8. Verfahren nach einem der vorgenannten Ansprüche, wobei die Zelle **(1)** eine sphärische Zelle ist.

9. Vorrichtung zur Erfassung der Strom-Spannungskurve einer mit einem spannungssensitiven Fluoreszenzfarbstoff dotierten Zelle **(1),** umfassend

- eine Kapillare **(2)** mit einem proximalen und einem distalen Ende sowie einen dazwischen liegenden konisch zulaufenden Bereich **(7),** dessen Innendurchmesser an mindestens einer Stelle kleiner ist als der Außendurchmesser der Zelle **(1),**
- eine mikrofluidische Vorrichtung, die in Kontakt mit dem proximalen Ende der Kapillare **(2)** steht, zum Positionieren der Zelle **(1)** in der Kapillare **(2),**
- eine erste Elektrode **(3),** die auf der proximalen Kapillarseite angeordnet ist,
- eine zweite Elektrode **(4),** die auf der distalen Kapillarseite angeordnet ist,
- ein Messinstrument zur Messung des zwischen den beiden Elektroden fließenden Gesamtstroms bei angelegter Spannung,

**dadurch gekennzeichnet, dass** die Vorrichtung ferner ein fluoreszenzoptisches Detektionssystem zur Messung des lokal und zeitlich aufgelösten Membranpotentials der Zelle **(1)** im elektrischen Feld **(5)** in Form eines optischen Signals und eine Auswertevorrichtung zur rechnerischen Auswertung der lokalen Ströme durch die Zellmembran mit den fluoreszenzoptisch gemessenen Membranpotentialen umfasst.

10. Vorrichtung nach Anspruch 9, wobei die Auswertevorrichtung ferner ein Modul zur Aufzeichnung der Strom-Spannungskurve umfasst.

11. Mikrofluidische Anordnung zur Messung der Strom-Spannungskurve von mindestens zwei Zellen **(1)** umfassend

- mindestens zwei Messvorrichtungen nach den Ansprüchen 9 bis 10, deren Kapillaren **(2)** parallel oder in Serie angeordnet sind,
- eine mikrofluidische Verteilervorrichtung, die die mindestens zwei Zellen **(1)** aus Zellsuspensionen in die jeweiligen Kapillaren **(2)** verteilt.

12. Mikrofluidische Anordnung nach Anspruch 11, wobei die mikrofluidische Verteilervorrichtung mindestens einen Zulauf für die Zellsuspension und Kanalstrukturen umfasst, die mit einer Druckströmung **(6)** beaufschlagbar sind, und dass ein Zulauf **(8)** und eine Kanalstruktur so miteinander in Kontakt stehen, dass die mindestens zwei Zellen **(1)** durch die Druckströmung **(6)** in die Kapillaren **(2)** verteilbar sind.

13. Mikrofluidische Anordnung nach einem der Ansprüche 11 oder 12, wobei die Anordnung rotierfähig positionierbar ist und die Flüssigkeit durch die Kanalstruktur in die Kapillaren **(2)** durch die Fliehkraft transportierbar ist.

**Claims**

1. Method for non-invasive measuring of the current-voltage curve of a cell (1), comprising the following steps:

a) provision of a measuring device comprising:

- a capillary (2) with a proximal and a distal end and an intermediate tapered area,
- a first electrode (3) arranged on the proximal capillary side,
- a second electrode (4) arranged on the distal capillary side,
- a fluorescence optical detection system for detecting a membrane potential of a cell (1) in the electric field (5) in the form of an optical signal,

b) provision of a cell (1) doped by means of a voltage-sensitive fluorescent dye,
c) insertion of the cell (1) to be measured into the lumen of the capillary (2) and fixing of the cell in the tapered area of the capillary (2) by means of a flow pressure (6) until an electric clamping resistance is formed between the distal and the proximal end of the capillary (2) by pressing the cell (1) against the capillary wall.
d) application of a voltage U between the two electrodes (3, 4) and recording the transcellular total current $I_{ges}$ (10) which flows through the capillary (2) blocked by the cell (1),
e) measuring of the locally and temporally resolved membrane potential $U_{lok}$ of the cell (1) in the electric field (5) between the electrodes (3, 4) using the fluorescence optical detection system,
f) calculation of a local current $I_{lok}$ using the measured transcellular total current $I_{ges}$ (10) from step d) and the measured local membrane potential $U_{lok}$ from step e),
g) repeating of steps step d) to f), wherein the voltage applied in step d) is being varied, and recording of the resulting local currents $I_{lok}$ against the local membrane potentials $U_{lok}$ from step e), whereby the current-voltage curve is obtained.

2. Method according to 1, wherein the calculation of the local current $I_{lok}$ in step f) is obtained by

    - allocating the local contribution of the current $I_{lok}$ to the respective measured local membrane potential $U_{lok}$ from step e) according to Ohm's law and
    - referencing to the transcellular total current $I_{ges}$ (10) by integrating or adding up the partial currents.

3. Method according to any one of the preceding claims, wherein the electric clamping resistance between the distal and proximal end of the capillary (2) is registered by means of impedance spectroscopy.

4. Method according to any one of the preceding claims, wherein the electric clamping resistance is between 50 and 10000 MOhms.

5. Method according to any one of the preceding claims, wherein the voltage applied between the electrodes (3, 4) is varied in pulses.

6. Method according to any one of the preceding claims, wherein the voltage-sensitive fluorescent dye is a membrane dye from the group of oxonols, styryles or hemicyanines.

7. Method according to claim 6, wherein the voltage-sensitive membrane dye is Annine-6.

8. Method according to any one of the preceding claims, wherein the cell (1) is a spherical cell.

9. Device for registering the current-voltage curve of a cell (1) doped with a voltage-sensitive fluorescent dye, comprising

    - a capillary (2) with a proximal and a distal end and an intermediate tapered area (7), the internal diameter of which is smaller than the outer diameter of the cell (1) in at least one place,
    - a micro-fluidic device, which is in contact with the proximal end of the capillary (2), for positioning the cell (1) in the capillary (2),
    - a first electrode (3) arranged on the proximal capillary side,
    - a second electrode (4) arranged on the distal capillary side,
    - a measuring instrument for measuring the total current flowing between the two electrodes when the voltage is applied,

**characterised in that** the device also comprises a fluorescence optical detection system for measuring the locally and temporally resolved membrane potential of the cell (1) in the electric field (5) in the form of an optical signal and an evaluation device for the computational evaluation of the local currents through the cell membrane with the membrane potentials measured by fluorescence optical methods.

10. Device according to claim 9, wherein the evaluation device also comprises a module for recording the current-voltage curve.

11. Micro-fluidic arrangement for measuring the current-voltage curve of at least two cells (1) comprising

    - at least two measuring devices according to claims 9 to 10, the capillaries (2) of which are arranged parallel or in series,
    - a micro-fluidic distribution device which distributes the at least two cells (1) from cell suspensions into the respective capillaries (2).

12. Micro-fluidic arrangement according to claim 11, wherein the micro-fluidic distribution device comprises at least one infeed for the cell suspension and channel structures which can be subjected to a pressure flow (6), and that *[sic]* an infeed (8) and a channel structure are in contact such that the at least two cells (1) can be distributed by the pressure flow (6) into the capillaries (2).

13. Micro-fluidic arrangement according to any one of claims 11 or 12, wherein the arrangement can be positioned so as to be capable of rotation and the fluid can be transported through the channel structure into the capillaries (2) by means of the centrifugal force.

**Revendications**

1. Procédé de mesure non invasive de la courbe intensité-tension d'une cellule (1) comprenant les étapes suivantes consistant à :

   a) prendre un dispositif de mesure ayant :

   - un capillaire (2) avec une extrémité proximale et une extrémité distale ainsi qu'une zone se rétrécissant suivant une forme conique dans l'intervalle,
   - une première électrode (3) installée sur le côté proximal du capillaire,
   - une seconde électrode (4) installée sur le côté distal du capillaire,
   - un système de détection de fluorescence optique pour détecter le potentiel de la membrane de la cellule (1) dans le champ électrique (5) sous la forme d'un signal optique,

   b) prendre une cellule (1) dopée par un colorant fluorescent sensible à la tension,
   c) introduire la cellule à mesurer (1) dans le lumen du capillaire (2) et fixer la cellule dans la zone allant en diminuant de manière conique du capillaire (2) par un écoulement sous pression (6) jusqu'à ce que la poussée de la cellule (1) contre la paroi du capillaire développe une résistance électrique de serrage entre l'extrémité distale et l'extrémité proximale du capillaire (2),
   d) appliquer une tension (U) entre les deux électrodes (3, 4) et enregistrer l'intensité totale transcellulaire $I_{ges}$ (10) passant dans le capillaire (2) bouché par la cellule (1),
   e) mesurer le potentiel de membrane $U_{lok}$ de la cellule (1) en résolution locale et dans le temps dans le champ électrique (5) entre les électrodes (3, 4) à l'aide du système de détection de fluorescence optique,
   f) calculer une intensité locale $I_{lok}$ à l'aide du courant total $I_{ges}$ (10) transcellulaire, mesuré, selon l'étape d) et du potentiel local $U_{lok}$ mesuré selon l'étape e),
   g) répéter les étapes d)-f), en modifiant la tension appliquée dans l'étape d) et enregistrer les intensités locales $I_{lok}$ résultantes par rapport aux potentiels locaux $U_{lok}$ de la membrane selon l'étape e) pour obtenir la courbe intensité-tension.

2. Procédé selon la revendication 1,
   **caractérisé en ce que**
   le calcul de l'intensité locale $I_{lok}$ dans l'étape f) se fait en :

   - appliquant la loi de Ohm pour la composante locale de l'intensité $I_{lok}$ et chaque potentiel de membrane, local $U_{lok}$, mesuré, selon l'étape e), et
   - relier à l'intensité totale transcellulaire $I_{ges}$ (10) en intégrant ou en additionnant les intensités partielles.

3. Procédé selon l'une des revendications précédentes,
   **caractérisé en ce que**
   la résistance de serrage électrique entre l'extrémité distale et l'extrémité proximale du capillaire (2) se détecte par spectroscopie d'impédance.

4. Procédé selon l'une des revendications précédentes,
   **caractérisé en ce que**
   la résistance de serrage électrique est comprise entre 50 et 10 000 Mohm.

5. Procédé selon l'une des revendications précédentes,
   **caractérisé en ce que**
   la tension appliquée entre les électrodes (3, 4) varie de manière impulsionnelle.

6. Procédé selon l'une des revendications précédentes,
   **caractérisé en ce que**
   le colorant fluorescent sensible à la tension est un colorant de membrane du groupe comprenant oxonole, styryle et hémicyanine.

7. Procédé selon la revendication 6,
   **caractérisé en ce que**
   le colorant de membrane, sensible à la tension, l'annine-6.

8. Procédé selon l'une des revendications précédentes,
   **caractérisé en ce que**
   la cellule (1) est une cellule sphérique.

9. Dispositif pour saisir la courbe intensité-tension d'une cellule (1) dopée avec un colorant fluorescent sensible à la tension comprenant :

   - un capillaire (2) avec une extrémité proximale et une extrémité distale ainsi qu'une zone intermédiaire conique (7) dont le diamètre intérieur à au moins un endroit est inférieur au diamètre extérieur de la cellule (1),
   - un dispositif micro-fluidique qui est en contact avec l'extrémité proximale du capillaire (2) pour positionner la cellule (1) dans le capillaire (2),
   - une première électrode (3) installée sur le côté proximale du capillaire,
   - une seconde électrode (4) installée sur le côté distal du capillaire,
   - un instrument de mesure pour mesurer le courant total passant entre les deux électrodes lorsque la tension est appliquée,

   dispositif **caractérisé en ce qu'**il comprend en outre
   un système de détection de fluorescence optique pour mesurer le potentiel de membrane à résolution locale et dans le temps de la cellule (1) dans le champ électrique (5) sous la forme d'un signal optique, et un dispositif d'exploitation pour exploiter par le calcul les intensités locales à travers la membrane de la cellule avec les potentiels de membrane, mesurés par fluorescence optique.

10. Dispositif selon la revendication 9,
    **caractérisé en ce que**
    le dispositif d'exploitation comporte en outre un module pour enregistrer la courbe intensité-tension.

11. Dispositif micro-fluidique pour mesurer la courbe intensité-tension d'au moins deux cellules (1) comprenant au moins deux dispositifs de mesure selon les revendications 9 à 10 dont les capillaires (2) sont en parallèle ou en série,

    - un dispositif de répartition micro-fluidique qui répartit au moins deux cellules (1) de suspension de cellules dans le capillaire respectif (2).

12. Dispositif micro-fluidique selon la revendication 11,
    **caractérisé en ce que**
    le dispositif de répartition micro-fluidique comporte au moins une entrée pour la suspension de cellules et des structures de canaux recevant un écoulement sous pression (6), et
    une arrivée (8) et une structure de canal sont en contact pour qu'au moins deux cellules (1) soient réparties par l'écoulement sous pression (6) dans les capillaires (2).

13. Dispositif micro-fluidique selon l'une des revendications 11 ou 12,
    **caractérisé en ce que**
    le montage est positionné de manière à pouvoir tourner et le liquide est transporté à travers la structure de canal dans les capillaires (2) par la force centrifuge.

## Fig. 1

## Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0980523 B1
- US 6268168 B
- US 6699697 B
- EP 1283985 B1
- EP 1178315 A

**In der Beschreibung aufgeführte Nicht-Patentliteratur**

- **P. FROMHERZ.** *BIOCHIMICA ET BIOPHYSICA ACTA,* 1993, vol. 1150, 111-122